# EUROPEAN PATENT APPLICATION

(11) **EP 4 799 618 A1**
(43) Date of publication of application: **02.09.2026**
(21) Application number: 24882959.0
(22) Date of filing: 24.10.2024
(51) Int. Cl.: A61K 31/351, A61K 31/495, A61K 45/06, A61P 9/00

(54) **COMBINATORY FORMULATION COMPRISING SGLT-2 INHIBITOR AND DPP-4 INHIBITOR FOR PREVENTING OR TREATING VASCULAR OR VALVULAR STENOSIS**

(30) Priority: 24.10.2023 KR 20230143222; 23.10.2024 KR 20240146113
(71) Applicant: Rednvia Co., Ltd., Seoul 05505 (KR)
(72) Inventor: KU, Il Whea, Seoul 05505 (KR); JO, Yong Hwa, Seoul 05505 (KR); KIM, Young Jae, Seoul 05505 (KR)
(74) Representative: Abel & Imray LLP
(86) International application number: PCT/KR2024/096387
(87) International publication number: WO 2025/089933

(57) **Abstract**

The present invention relates to a pharmaceutical composition for the prevention or treatment of vascular or valvular stenosis, comprising a gliflozin-based drug and a gliptin-based drug. When the gliflozin-based drug and the gliptin-based drug according to the present invention are administered together, calcification is synergistically reduced compared to when each is administered alone, and therefore, the composition according to the present invention is very useful for vascular or valvular stenosis.

## Description

### BACKGROUND OF THE INVENTION

### 1. Field of the Invention

The present invention relates to a pharmaceutical composition for the prevention or treatment of vascular or valvular stenosis, comprising an SGLT-2 (sodium-glucose cotransporter 2) inhibitor, such as a drug of the gliflozin family, and a DPP-4 (dipeptidyl peptidase-4) inhibitor.

### 2. Description of the Related Art

Aortic valve stenosis is a disorder or condition in which the aortic valve, the valve at the site where blood flows from the left ventricle to the aorta, does not open properly when the left ventricle contracts. The pathophysiology of aortic valve stenosis is complicated and involves multiple pathological processes such as lipid retention, oxidation, chronic inflammation, fibrosis, and calcification. In particular, the symptom or disorder in which calcification occurs in the aortic valve is known as calcific aortic valve disease (CAVD). There are currently no approved drug treatments specific to aortic valve stenosis or CAVD. Therefore, the only treatment options are surgical aortic valve implantation (SAVI) and transcatheter aortic valve implantation (TAVI) via non-surgical catheterization.

Cardiovascular calcification contributes to the worsening of hypertension, heart failure, acute coronary syndrome, and valvular disease, and causes various complications. Calcification may occur independently or together with fibrosis. A number of epidemiologic studies have shown that vascular calcification independently increases mortality. Vascular calcification occurs by a mechanism similar to the normal fetal or post-fracture osteogenic program and is activated in old age, diabetes, chronic renal failure, and chronic inflammatory diseases. Inflammatory responses promote mineral loss from bones, and free minerals are phagocytosed by abnormal vascular endothelial cells. On the other hand, calcification also occurs in the valves of the heart, and calcification in the heart valves, especially in the aortic valve, leads to narrowing of the aortic valve, i.e., aortic valve stenosis.

The calcification process in the aortic valve, which leads to aortic valve stenosis, begins deep in the aortic valve tissue, near the margin of the attachment site. In advanced disease, the nodules extend through the surface of the valve stenosis. The early stage of the calcification process is called aortic valve sclerosis, and the later stage, aortic valve stenosis, occurs when the functional valve area is sufficiently reduced to cause blood flow impairment.

There are currently no non-invasive treatments available to cure CAVD.

However, US Patent No. 9416196 discloses that DPP-4 (dipeptidyl peptidase-4) inhibitors such as gliptin can treat aortic valve calcification. DPP-4 (dipeptidyl peptidase-4), also known as CD26 (cluster of differentiation 26), is a protein known to be involved in immune regulation, apoptosis, and signal transduction. According to the above-mentioned US patent, when blood vessels and valves are calcified, the expression of DPP-4 increases, and when a DPP-4 inhibitor is administered, the calcification is significantly reduced, so it is disclosed that a DPP-4 inhibitor can be usefully used for the treatment or prevention of blood vessel or valve diseases. As DPP-4 inhibitors, several types of gliptin family drugs have been disclosed, including sitagliptin, vildagliptin, saxagliptin, linagliptin, dutogliptin, gemigliptin, alogliptin, anagliptin, and evogliptin.

Meanwhile, gliflozin is a general term for a group of drugs that are widely used as SGLT-2 (sodium-glucose cotransporter 2) inhibitors to treat type 2 diabetes, and specifically, dapagliflozin, ertugliflozin, empagliflozin, canagliflozin, bexagliflozin, tofagliflozin, ipragliflozin, enavogliflozin, and luseogliflozin have been developed. SGLT-2 inhibitors are drugs that inhibit the reabsorption of sodium and glucose in the renal tubules, increasing their excretion from the body. Recently, WO 2021/037400 A discloses that dapagliflozin is effective in heart failure with reduced ejection fraction.

However, no drug has yet been developed to treat stenosis caused by calcification of blood vessels or valves. The present inventors have made great efforts to develop a drug for the prevention or treatment of such stenosis. As a result, the inventors have found that the combination of gliflozin and gliptin has a synergistic effect in inhibiting calcification of blood vessels or valves compared to gliptin or gliflozin alone, and thus completed the present invention.

### SUMMARY OF THE INVENTION

It is an object of the present invention to provide a novel composition or method for the prevention or treatment of vascular or valvular stenosis.

To achieve the above object, in an aspect of the present invention, the present invention provides a pharmaceutical composition for the prevention or treatment of vascular or valvular stenosis comprising an SGLT-2 inhibitor and a DPP-4 inhibitor.

### ADVANTAGEOUS EFFECT

A pharmaceutical composition according to the present invention enables the prevention or treatment of vascular or valvular stenosis.

### BRIEF DESCRIPTION OF THE DRAWINGS

Figure 1A is a set of photographs showing the extent of calcification when calcification-induced aortic smooth muscle cells were treated with different concentrations of the gliptin family drugs, which are DPP-4 inhibitors.
Figure 1B is a graph showing the extent of calcification quantified by analyzing the results of Figure 1a through absorbance analysis.
Figure 2A is a set of photographs showing the extent of calcification when calcification-induced aortic smooth muscle cells were treated with different concentrations of the gliflozin family drugs, which are SGLT-2 inhibitors.
Figure 2B is a graph showing the extent of calcification quantified by analyzing the results of Figure 2a through absorbance analysis.
Figure 3A is a set of photographs comparing the degree of inhibition of calcification when evogliptin and dapagliflozin were treated alone and in combination in calcification-induced aortic smooth muscle cells.
Figures 3B and 3C are graphs showing the extent of calcification quantified by analyzing the results of Figure 3a through absorbance analysis.
Figure 4A is a set of photographs comparing the degree of inhibition of calcification when evogliptin and empagliflozin were treated alone and in combination in calcification-induced aortic smooth muscle cells.
Figure 4B is a graph showing the extent of calcification quantified by analyzing the results of Figure 4a through absorbance analysis.
Figure 5A is a set of photographs comparing the degree of inhibition of calcification when evogliptin and canagliflozin were treated alone and in combination in calcification-induced aortic smooth muscle cells.
Figure 5B is a graph showing the extent of calcification quantified by analyzing the results of Figure 5a through absorbance analysis.
Figure 6A is a set of photographs comparing the degree of inhibition of calcification when evogliptin and ertugliflozin were treated alone and in combination in calcification-induced aortic smooth muscle cells.
Figure 6B is a graph showing the extent of calcification quantified by analyzing the results of Figure 6a through absorbance analysis.
Figure 7A is a set of photographs comparing the degree of inhibition of aortic valve calcification when evogliptin and dapagliflozin were administered alone and in combination in a mouse model in which calcification was induced by administering vitamin D3.
Figure 7B is a graph showing the extent of aortic valve calcification in samples administered with evogliptin and dapagliflozin alone and in combination, respectively.
Figure 8A is a graph showing the extent of renal calcification in samples administered with evogliptin and dapagliflozin alone and in combination, respectively.
Figure 8B is a graph showing the extent of vascular calcification in samples administered with evogliptin and dapagliflozin alone and in combination, respectively.

### DESCRIPTION OF THE PREFERRED EMBODIMENTS

Hereinafter, the present invention is described in detail.

The embodiments of this invention can be modified in various other forms, and the scope of the present invention is not limited to the embodiments described below. It is well understood by those in the art who has the average knowledge on this field that the embodiments of the present invention are given to explain the present invention more precisely.

In addition, the "inclusion" of an element throughout the specification does not exclude other elements, but may include other elements, unless specifically stated otherwise.

In an aspect of the present invention, the present invention provides a combination of an SGLT-2 inhibitor and a DPP-4 inhibitor for the prevention or treatment of vascular or valvular stenosis.

In another aspect of the present invention, the present invention provides a pharmaceutical composition for the prevention or treatment of vascular or valvular stenosis, comprising an SGLT-2 inhibitor and a DPP-4 inhibitor.

In another aspect of the present invention, the present invention provides a health functional food composition for the prevention or amelioration of valvular stenosis, comprising an SGLT-2 inhibitor and a DPP-4 inhibitor.

In another aspect of the present invention, the present invention provides a method for preventing or treating vascular or valvular stenosis by administering the SGLT-2 inhibitor and DPP-4 inhibitor to a subject in need thereof in an amount effective for the prevention or treatment of vascular or valvular stenosis.

In another aspect of the present invention, the present invention provides a combination comprising the SGLT-2 inhibitor and DPP-4 inhibitor for the preparation of a medicament for preventing or treating vascular or valvular stenosis.

In another aspect of the present invention, the present invention provides a combination comprising the SGLT-2 inhibitor and DPP-4 inhibitor for use in preventing or treating vascular or valvular stenosis.

In another aspect of the present invention, the present invention provides a combination preparation comprising the SGLT-2 inhibitor and DPP-4 inhibitor for use in the prevention or treatment of vascular or valvular stenosis.

In this specification, the SGLT-2 inhibitor includes drugs of the gliflozin family, and the DPP-4 inhibitor includes drugs of the gliptin family. In this specification, gliflozin family drugs may be used to mean SGLT-2 inhibitors, and DPP-4 inhibitors may be used to mean gliptin family drugs.

Throughout this specification, the term "combination" refers to a clinical treatment of administering a gliflozin family drug and a gliptin family drug simultaneously or at different times for the purpose of preventing or treating vascular or valvular stenosis. The compositions, methods, uses, combinations and combination preparations in this specification all presuppose combination use. The possible specifics of combination use are described below. Combination preparations include both combinations of each individual preparation and complex preparations.

The following description applies to all forms of the present invention, including the compositions, methods, uses, combinations and combination preparations.

The blood vessel includes all blood vessels in the body, specifically cardiovascular vessels associated with the heart, and more specifically may include the aorta, the vena cava, the pulmonary artery, the vena cava, and the renal artery. The valve is a cardiac valve, including an aortic valve, a pulmonary valve, a mitral valve, and a tricuspid valve, but preferably refers to an aortic valve. The stenosis may occur in patients suffering from cardiovascular diseases such as heart failure and arteriosclerosis, or renal diseases such as chronic renal failure.

The above vascular or valvular stenosis may be accompanied by calcification of the vessels or valves. Vascular calcification occurs in patients with cardiovascular diseases such as heart failure, arteriosclerosis, or atherosclerosis, or in patients with renal diseases such as renal arteriosclerosis or renal artery atherosclerosis. Renal disease includes chronic renal disease.

Vascular calcification or vascular stenosis, which occurs in patients with cardiovascular or renal disease, can occur in any blood vessel in the body.

In the present invention, calcification refers to a symptom in which calcium is deposited in the heart, lungs, kidneys, blood vessels, or valves, causing the tissue to harden. As calcification progresses, calcification in the kidneys leads to renal disease, narrowing of blood vessels or valves, and calcification in the aortic valve leads to aortic valve stenosis.

Calcification includes medial calcification or atherosclerotic calcification. Calcified tissue is called calcific. Intimal calcification occurs in association with atherosclerosis. Atherosclerosis begins with the accumulation of fat-rich macrophages and T lymphocytes on the inner side of blood vessels or valves, forming a fatty layer, followed by the migration of smooth muscle cells from the media. The chemodynamic substances that stimulate their movement are thought to be produced in nearby endothelium, activated phagocytic cells, etc.

The migrated smooth muscle cells proliferate, accumulate fat, and produce extracellular matrix. Calcification occurs in the center of the atherosclerotic plaque.

Medial calcification occurs independently of atherosclerosis and intimal calcification. Medial calcification in the peripheral arteries is called Muckeberg's sclerosis and is most commonly observed in older diabetic patients. It is known that smooth muscle cells and elastin are involved in the development of calcification.

It has been reported that calcification caused by renal dysfunction is mainly medial calcification that occurs in the media, whereas calcification caused by atherosclerosis or atherosclerosis-related calcification is mainly reported to occur in the intima.

In addition, in patients with renal disease, more specifically, in patients with chronic renal failure or chronic renal disease, calcification of the kidney itself occurs, and thus, the composition of the present invention can be used to alleviate or treat chronic renal disease, more specifically, chronic renal disease accompanied by calcification.

The treatable stenosis in the present invention is not particularly limited, but includes stenosis clinically diagnosed as stenosis, and may include an early stage of hardening that is not clinically diagnosed as stenosis. It may include a stage at which symptoms of fibrosis and/or calcification are identified, and preferably the stenosis may include a stage at which symptoms of calcification are identified. In the present invention, "treatment" includes reducing the degree of stenosis in the tissue to be treated, thereby slowing the progression of stenosis or reversing the symptoms of stenosis to a normal state. When it is said that the progression of stenosis is slowed, it means that the progression of stenosis is significantly slowed down as a relative concept, compared to when the combination treatment according to the present invention is not performed, for example, when no treatment is performed or only a general gliflozin family drug or gliptin family drug is administered alone. Slowing the progression of stenosis can mean, for example, slowing it down by 1%, 2%, 3%, 5%, 10%, 15%, 20%, 30%, 40%, or 50%, and the degree of stenosis progression can be quantitatively assessed, for example, through blood flow velocity, degree of calcification, etc.

When stenosis is accompanied by calcification, the treatment includes not only reversing the calcification to normal completely and making it normal tissue, but also slowing the progression of calcification and reducing the extent of calcification that has already occurred. When it is said that the progression of calcification is slowed, it means that the progression of calcification is significantly slowed down as a relative concept, compared to when the combination treatment according to the present invention is not performed, for example, when no treatment is performed or only a general gliflozin family drug or gliptin family drug is administered alone. Slowing the progression of calcification includes, for example, slowing the rate of increase in the amount of calcium deposited, or the area or weight of calcified tissue, as an indicator of calcification, by more than 1%, more than 2%, more than 3%, more than 5%, more than 10%, more than 15%, more than 20%, more than 30%, more than 40%, or more than 50%. Reducing the extent of calcification includes reducing the area of calcified tissue by 1% or more, 2% or more, 3% or more, 5% or more, 10% or more, 15% or more, 20% or more, 30% or more, 40% or more, or 50% or more, based on the area of calcified tissue before starting the combined administration of the gliflozin family drug and the gliptin family drug according to the present invention.

A method for quantifying the area of calcified tissue can be used, for example, by quantifying the stained area observed by staining with Alizarin Red S (ARS) or von Kossa (VK) for calcium deposition. In the present invention, "preventing" may include alleviating calcification before it occurs or preventing its worsening during its progression.

In the present invention, the gliflozin family drug is not specifically limited to SGLT-2 inhibitors, but may include one or more gliflozin family drugs selected from the group consisting of dapagliflozin, ertugliflozin, empagliflozin, canagliflozin, bexagliflozin, tofagliflozin, ipragliflozin, enavogliflozin, and luseogliflozin. The gliptin family drug is not specifically limited to DPP-4 inhibitors, but may include one or more gliptin family drugs selected from the group consisting of evogliptin, linagliptin, saxagliptin, and sitagliptin.

In a specific embodiment of the present invention, the gliflozin family drug may be dapagliflozin, ertugliflozin, empagliflozin or canagliflozin, and in another specific embodiment, it may be dapagliflozin.

In a specific embodiment of the present invention, the gliptin family drug may be evogliptin, linagliptin, sitagliptin or saxagliptin, and in another specific embodiment, it may be evogliptin
In the present invention, a drug of the gliflozin family and a drug of the gliptin family may be included in a composition or administered in a therapeutically effective amount to prevent calcification of a blood vessel or valve. The pharmaceutical composition or health functional food composition according to the present invention includes not only being provided in a formulation for a single administration in which the gliflozin family drug and the gliptin family drug are physically mixed, but also being prepared in a formulation for separate administration and then combined upon administration.

It is desirable that the administration dosage of the gliflozin family drug and the gliptin family drug be determined so that they are administered in a therapeutically effective amount within a range that is not harmful to the subject of administration.

The dosage of these drugs can be determined according to weight, age, gender, health condition, diet, administration frequency, administration method, excretion and severity of a disease, and a person skilled in the art can determine an appropriate amount. For example, each of the gliflozin family drug and the gliptin family drug can be administered at a dose of 0.01 to 500 mg per day, and can be administered once or several times per day, for example, twice, three times, or four times.

And, their administration cycle can be determined daily, in a cycle of days selected from 2 to 7 days, or in an irregular cycle. The duration of administration can be from 2 days to 1 month in the short term, from more than 1 month to 3 months in the medium term, from more than 3 months to 1-3 years in the long term, and even for the remaining lifespan. It is clear that the dosage and administration cycle may vary depending on the number of days of administration.

The dosage ratio of the gliflozin drug and the gliptin drug can be determined by a person skilled in the art as a therapeutically effective amount, and for example, can be determined within a range of 1:1000 to 1000:1, 1:500 to 500:1, 1:100 to 100:1, 1:50 to 50:1, 1:10 to 10:1, 1:5 to 5:1, and 1:2 to 2:1 for a certain period of time based on the weight of the active ingredients.

In the present invention, the gliflozin family drug and the gliptin family drug may be administered in combination at the same time or at different times. When it is said to be administered at the same time, it does not only include co-administration in a physically mixed form, but also may include administration at different times within a range of 6 hours, 5 hours, 4 hours, 3 hours, 2 hours, or 1 hour where drug-drug interactions may be possible. The pharmaceutical composition or health functional food composition according to the present invention includes not only being provided in a formulation for a single administration in which the gliflozin family drug and the gliptin family drug are physically mixed, but also being prepared in a formulation for separate administration and then combined upon administration. When the compositions of the present invention are provided as separate formulations, they may be administered simultaneously or at different times as described above. The term "pharmaceutical composition" in the present invention is not intended to have a limited meaning to refer to a physically mixed composition, but rather to indicate use as a medicament.

Even if the drugs are administered at different times, the effect of co-administration of the drugs can be achieved by adjusting the blood drug profile to allow the gliflozin drug and the gliptin drug to interact in the blood, and for example, they can be administered at different times in a range of more than 6 hours to within 48 hours.

For co-administration, the gliflozin drug and the gliptin drug may be formulated and used independently of each other. It is also possible to use commercialized preparations that contain each drug as an active ingredient for vascular or valvular stenosis as well as other indications.

For co-administration, the active ingredients of the gliflozin family drugs and the gliptin family drug can be formulated together as a combination preparation to include both in one formulation.

When the drugs are formulated independently or as a combination preparation, the preparations may be formulated for oral administration, such as tablets, capsules, powders, granules or suspensions, or may be formulated in the form of injections that can be administered parenterally, but is preferably formulated for oral administration. When formulated, these preparations may further contain one or more pharmaceutically acceptable additives. These additives may include at least one selected from the group consisting of excipients (diluents), binders, disintegrants, lubricants, and colorants.

The composition according to the present invention can be administered alone or treated together with surgical operation, hormone therapy, drug therapy and biological regulators for the prevention or treatment of vascular or valvular calcification.

For co-administration, a health functional food composition containing both an active ingredient of the gliflozin family drug and an active ingredient of the gliptin family drug can be formulated.

There are no specific limitations on the other ingredients that may be included in the health functional food composition of the present invention, which may include, for example, various herbal extracts, food additives, or natural carbohydrates as additional ingredients, such as those found in conventional foods. The composition may further include food additives, such as flavoring agents, coloring agents, fillers, stabilizers, and the like, which are commonly used in the art.

It is also clear that in the health functional food composition, the gliflozin family drug and the gliptin family drug are provided as separate preparations and then used together.

For the above health functional food composition, the dosage, administration cycle, and administration days can all be applied as described above.

Hereinafter, the present invention will be described in detail by the following examples.

However, the following examples are only for illustrating the present invention, and the contents of the present invention are not limited thereto.

### Example 1: Cell based in vitro assay

### Experimental methods:

1. Human aortic smooth muscle cells (HASMCs) were seeded in 48-well plates at 1x10⁴ cells per well.
2. The cells were cultured for 3 days until the cell density reached approximately 80%.
3. After 3 days, the cell density was checked to see if it was about 80%.
4. An osteogenesis medium (OM) was prepared to induce calcification in HASMCs. The composition of the medium was prepared to contain 10% FBS and 1% penicillin-streptomycin and 3 mM CaCl₂ in DMEM (Dulbecco's Modified Essential Medium) High glucose.
5. Solutions were prepared by adding DPP-4 inhibitors (evogliptin, sitagliptin, linagliptin, saxagliptin) and SGLT-2 inhibitors (dapagliflozin, canagliflozin, empagliflozin, ertugliflozin) to the OM prepared above, either alone or in combination, at different concentrations.

For gliptin alone, the drugs were treated at concentrations of 10, 25, 50, and 100 µM (except for saxagliptin, which was treated at concentrations of 2, 5, 10, and 20 µM), and for gliflozin alone, the drugs were treated at concentrations of 10, 20, 30, and 40 µM.

To compare the effects of combined treatment, evogliptin alone was treated at concentrations of 10 and 25 µM, dapagliflozin alone was treated at concentrations of 1, 5, 10, 20, 30, and 40 µM, and the other gliflozin drugs except dapagliflozin were treated at concentrations of 10, 20, 30, and 40 µM, and combined treatment was performed with each concentration combination.
6. Drug solutions were treated to wells appropriate for each group.
7. Every other day, a solution containing the drug in OM was prepared and treated to the wells.
8. After 12 days of treatment with the drug solution, Alizarin Red staining was performed to determine the extent of calcification.
9. For a quantitative comparative analysis of the extent of calcification, 10% cetylpyridinium chloride Solution was dispensed into each well.
10. The reaction was carried out on a shaker for 30 minutes to allow the Alizarin Red Solution to be eluted from each well.
11. The absorbance was measured at a wavelength of 540 nm using a reader to compare the degree of calcification. The results were normalized based on OM without any drug treatment as a control.

### Results:

The results of single and combined treatment with each of the gliptin family drugs and gliflozin family drugs are presented in figures.

In the figures, Evo indicates evogliptin, Lina indicates linagliptin, Sita indicates sitagliptin, Saxa indicates saxagliptin, Dapa indicates dapagliflozin, Empa indicates empagliflozin, Cana indicates canagliflozin, Ertu indicates ertugliflozin, and OM indicates the control with no drug treatment in the medium in which the cells were cultured.

The absorbance was measured and normalized to the control along with the staining results.

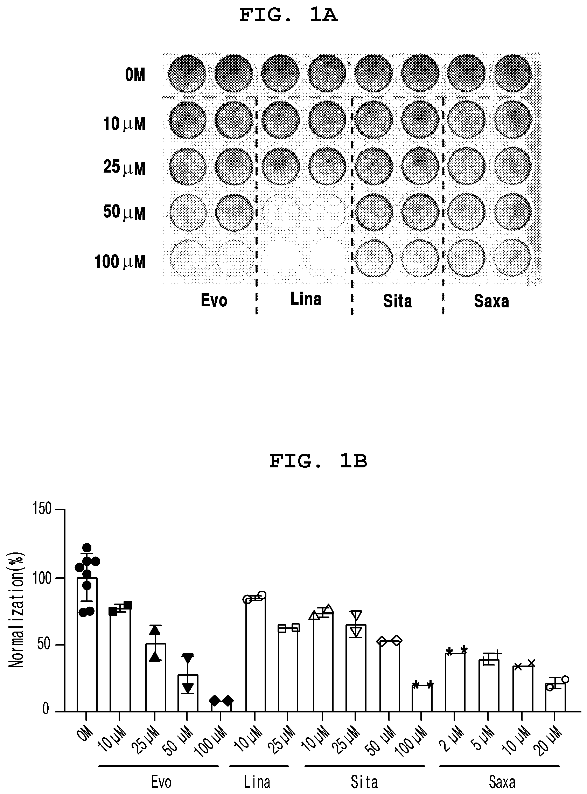

Figures 1A and 1B show the results when the gliptin family drugs, which are DPP-4 inhibitors, were treated at each concentration. All of them were confirmed to inhibit calcification in a concentration-dependent manner.

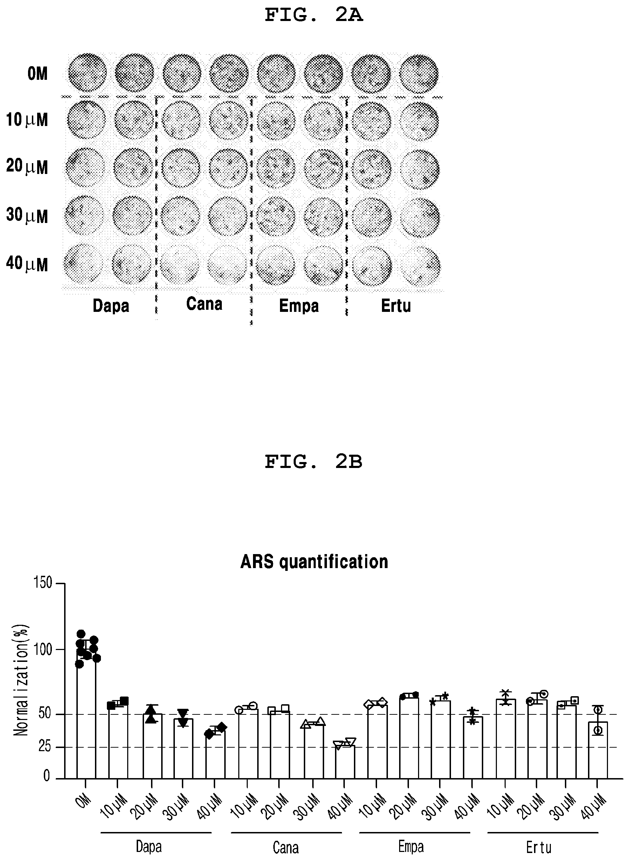

Figures 2A and 2B show the results when the gliflozin family drugs, which are SGLT-2 inhibitors, were treated at each concentration. All of them were confirmed to inhibit calcification in a concentration-dependent manner.

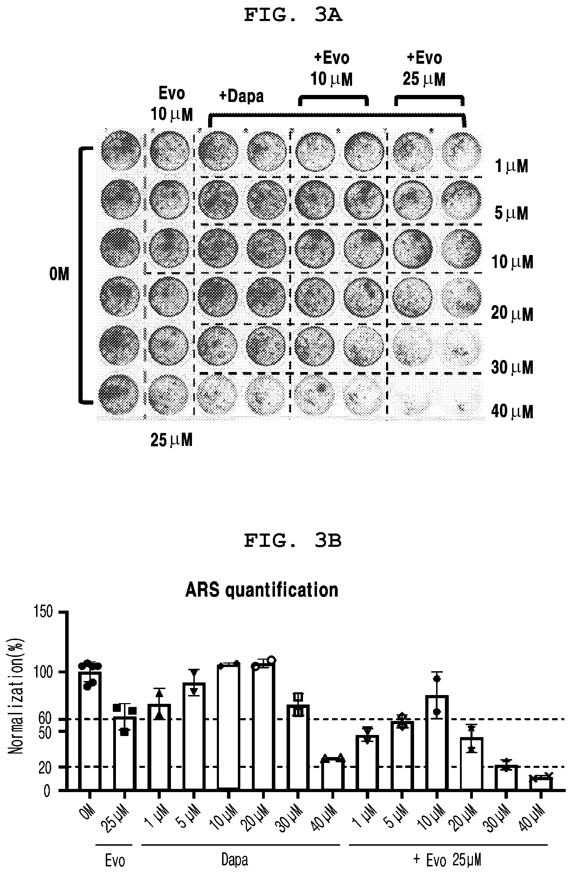

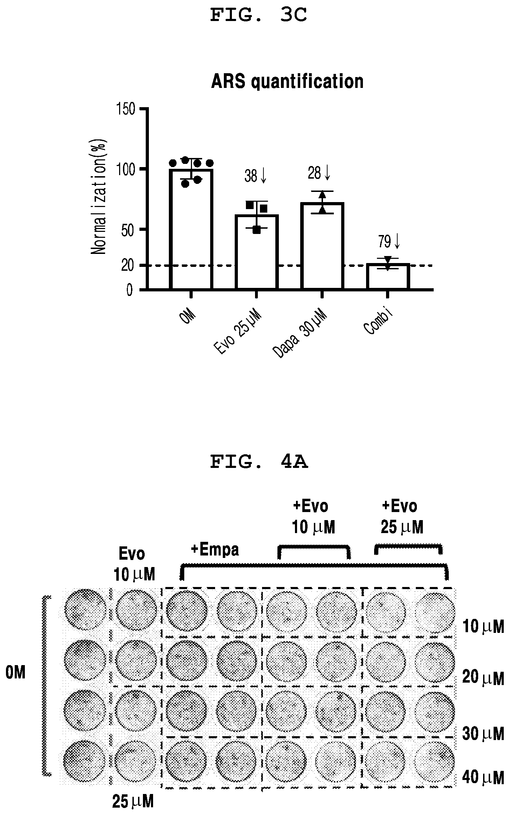

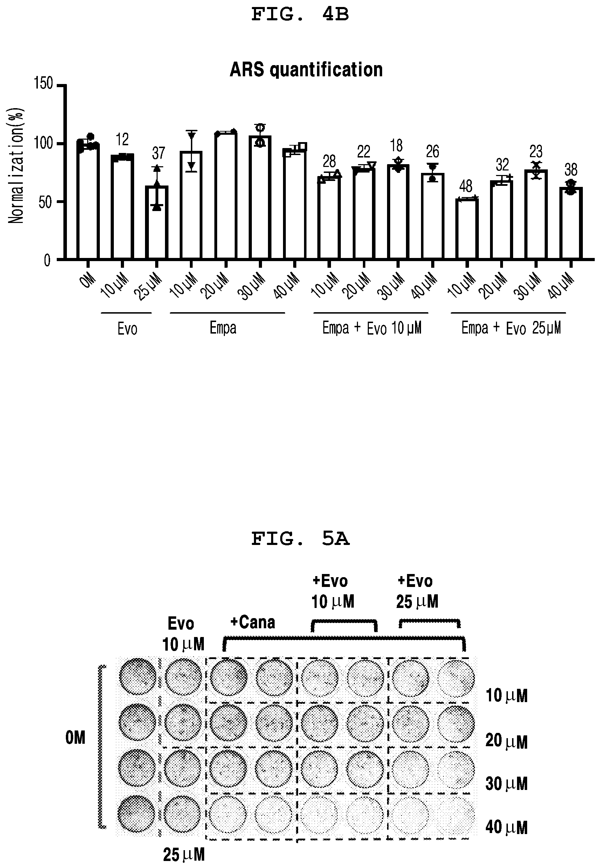

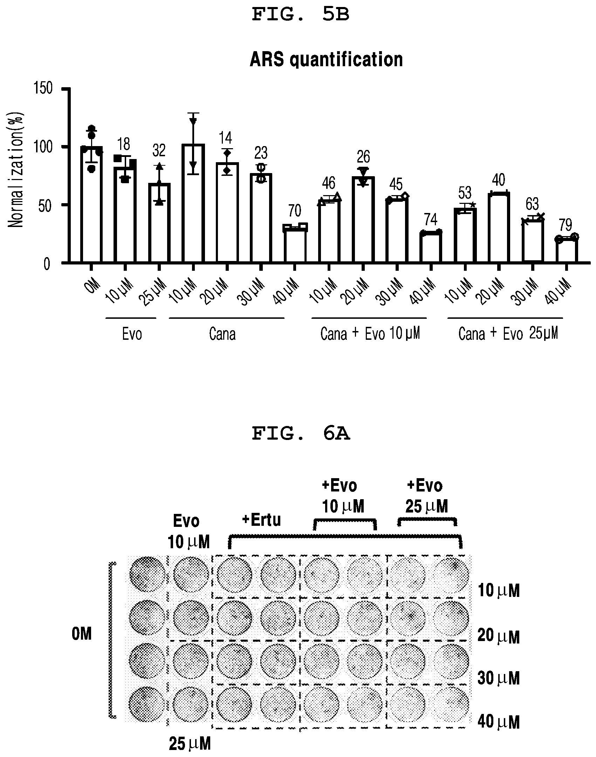

Figures 3A to 6B show the degree of inhibition of calcification when DPP-4 inhibitors and SGLT-2 inhibitors were treated alone and in combination.

When evogliptin and dapagliflozin were treated together, a synergistic anti-calcification effect was confirmed compared either treatment alone.

When evogliptin and empagliflozin were treated together, a synergistic anti-calcification effect was confirmed compared either treatment alone.

When evogliptin and canagliflozin were treated together, a synergistic anti-calcification effect was confirmed compared either treatment alone.

When evogliptin and ertugliflozin were treated together, a synergistic anti-calcification effect was confirmed compared either treatment alone.

### Example 2: In vivo mouse model assay

### Experimental methods:

### 1. Preparation of vitamin D3 solution

A vitamin D3 solution was obtained by dissolving the exact weight of vitamin D3 in a solution of 30% PEG400, 0.5% Tween80, 5% propylene glycol, and 64.5% normal saline (6.5X10⁴ IU/ml (1.625 mg/ml)) by sonication for 1 hour.

### 2. Preparation of gliptin and gliflozin

All materials were accurately weighed and dissolved or dispersed in 0.5% CMC sodium salt to prepare evogliptin at 1 mg/kg/100 µl and dapagliflozin at 2 mg/kg/100 µl.

### 3. Preparation of animal (mouse)

Animal: C57BL/6 mouse (OrientBio, Sungnam-city, Korea)
Gender: male
Age on arrival: C57BL/6 (6 weeks)
Raising: Animals were raised in filter cap polycarbonate cages, 10 animals each, in a temperature (25°C) and humidity (45-55%) controlled sterile clean room. The light:dark cycle was 12 hours:12 hours, and sterilized food and water were provided with free access.
Guidelines: All animals were treated in accordance with the Daegu Haany University Institutional Animal Care and Use Committee (Gyeongsan, Korea).

### 4. In vivo drug administration: A mouse model of calcification induced by vitamin D administration

After acclimation for one week, the mice were randomly divided into 18 groups.

Mice in all groups were administered with vitamin D3 (6.5X10⁵ IU/kg) via subcutaneous injection every 24 hours for 3 days. At the same time, 0.5% CMC was administered orally to groups except the non-drug treated group (Disease), and gliptin and gliflozin were administered orally at 1-hour intervals, individually or in combination, according to the concentration. Seven days later, the intact heart, kidney, and aorta were excised and isolated under anesthesia. The excised heart was preserved in buffered formalin until further histopathological and histomorphologic examination.

### 5. Aortic valve calcification analysis

Individual heart samples were cut crosswise, one section at a time, around the origin/root region of the ascending aorta. Histological sections were then prepared from all 180 samples received. All cross-sectioned cardiac-aortic valve sections were refixed in 10% neutral buffered formalin for 24 hours to prepare histological samples. After the samples were embedded in paraffin, two serial sections of 3 to 4 µm thickness were prepared on each paraffin block and stained with hematoxylin & eosin (HE) for general histopathology or von Kossa (VK) for calcium deposition, respectively.

In histopathologic analysis, the mean calcified aortic valve area (%/mm²) was calculated from VK staining. The histopathologist was blinded to group distribution when this analysis was performed. Histopathological examinations of one section, cross-section, histological area of each cardiac-aortic valve, and a total of 180 C57BL/6 mouse heart samples from each group were statistically analyzed.

### 6. Analysis of renal calcification and aortic calcification

The individual kidney samples were analyzed with the left kidney, and the aorta was analyzed with the descending aorta. The excised left kidney and descending aorta were each diluted with cooled physiological saline to 10 times the tissue volume, homogenized using a homogenizer, and stored in 0.6 N HCl at 4 °C for 24 hours to separate calcium from the tissue. Then, the supernatant was separated using a refrigerated centrifuge, and the amount of calcium in the supernatant was measured. For accurate measurement, quantification was performed based on the amount of protein.

### Results:

The results of single and combined treatment with each of the gliptin family drugs and gliflozin family drugs are presented in figures.

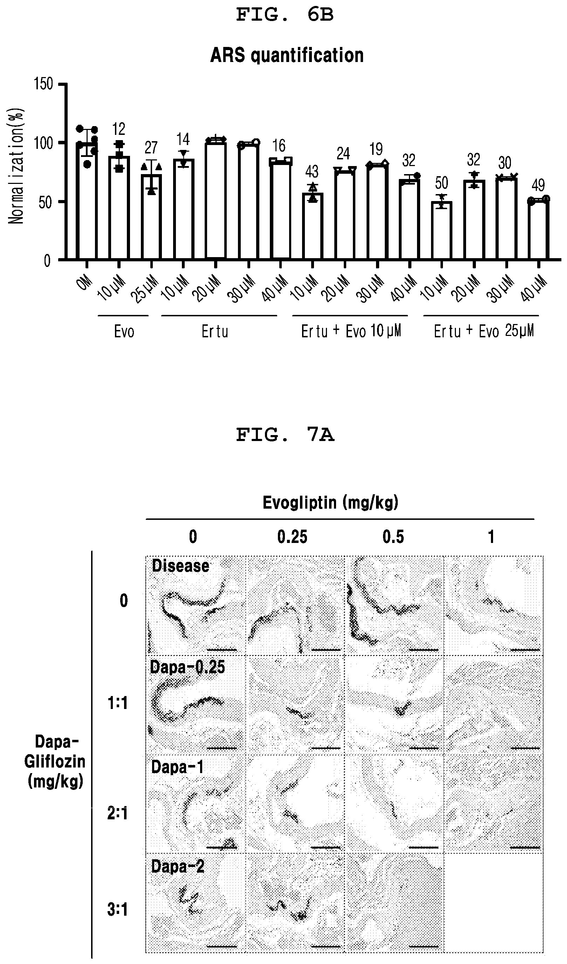

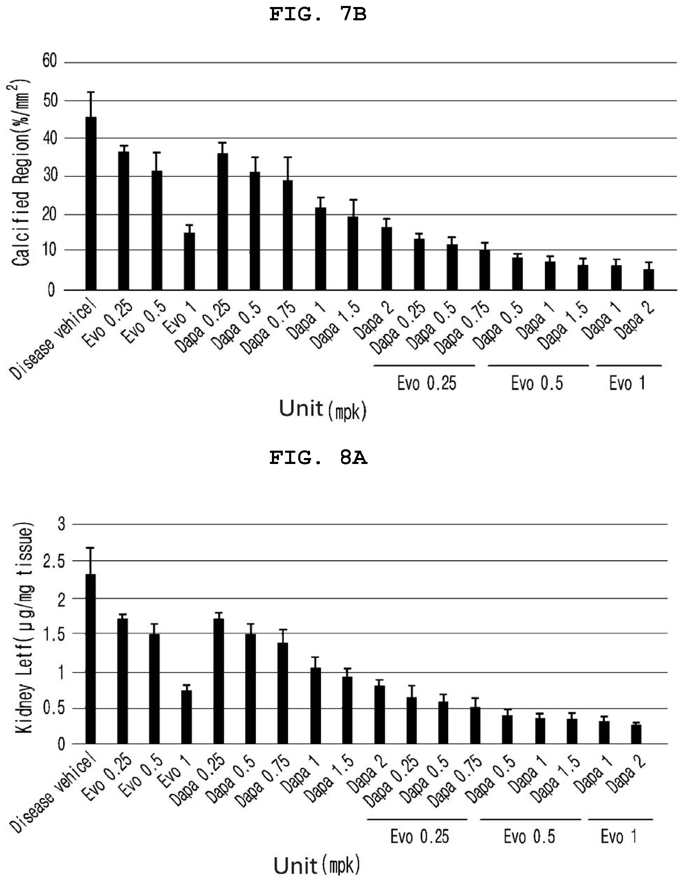

Figures 7A and 7B show the degree of inhibition of aortic valve calcification when evogliptin and dapagliflozin were administered alone and in combination in a mouse model of calcification induced by administration of vitamin D3. When evogliptin and dapagliflozin were treated together, a synergistic anti-calcification effect was confirmed compared either treatment alone.

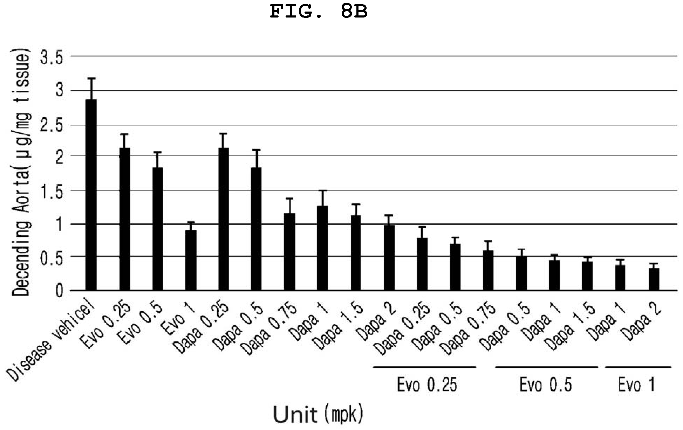

Figures 8A and 8B show the degree of inhibition of renal and aortic calcification when evogliptin and dapagliflozin were administered alone and in combination in a mouse model of calcification induced by administration of vitamin D3.

As shown in the results of aortic valve calcification, when evogliptin and dapagliflozin were treated together, a synergistic anti-calcification effect was confirmed compared either treatment alone.

## Claims

1. A pharmaceutical composition for the prevention or treatment of renal disease or vascular or valvular stenosis, comprising a gliflozin family drug and a gliptin family drug.

2. The pharmaceutical composition according to claim 1, wherein the valve includes an aortic valve.

3. The pharmaceutical composition according to claim 1, wherein the vascular or valvular stenosis is accompanied by calcification of the blood vessel or valve, and the renal disease is accompanied by calcification of the kidney.

4. The pharmaceutical composition according to claim 3, wherein the calcification of the blood vessel or valve is caused by arteriosclerosis or atherosclerosis.

5. The pharmaceutical composition according to claim 3, wherein the calcification of the blood vessel or kidney occurs in patients with renal disease.

6. The pharmaceutical composition according to claim 1, wherein the aortic valve stenosis includes calcific aortic valve disease (CAVD).

7. The pharmaceutical composition according to claim 1, wherein the gliflozin family drug is at least one selected from the group consisting of dapagliflozin, ertugliflozin, empagliflozin, canagliflozin, bexagliflozin, tofagliflozin, ipragliflozin, enavogliflozin, and luseogliflozin.

8. The pharmaceutical composition according to claim 1, wherein the gliptin family drug is at least one selected from the group consisting of evogliptin, linagliptin, saxagliptin and sitagliptin.

9. The pharmaceutical composition according to claim 1, wherein the gliflozin family drug is any one selected from the group consisting of dapagliflozin, ertugliflozin, empagliflozin, canagliflozin, bexagliflozin, tofagliflozin, ipragliflozin, enavogliflozin, and luseogliflozin, and the gliptin family drug is any one selected from the group consisting of evogliptin, linagliptin, saxagliptin, and sitagliptin.

10. The pharmaceutical composition according to claim 1, wherein the gliflozin family drug and the gliptin family drug are administered together.

11. The pharmaceutical composition according to claim 1, wherein the gliflozin family drug and the gliptin family drug are administered simultaneously or at different times.

12. The pharmaceutical composition according to claim 1, wherein the composition is provided as a combination preparation.

13. A combination preparation for preventing or treating renal disease or vascular or valvular stenosis, comprising a composition containing a gliflozin family drug and a composition containing a gliptin family drug, wherein each composition is used in combination.

14. A method for preventing or treating renal disease or vascular or valvular stenosis by administering a gliflozin family drug and a gliptin family drug to a subject in need of prevention or treatment of renal disease or vascular or valvular stenosis in an amount effective for the prevention or treatment.

15. The method according to claim 14, wherein the gliflozin family drug and the gliptin family drug are administered simultaneously or at different times.
